# FASCICULE DE BREVET EUROPEEN

(11) **EP 2 408 469 B1**
(45) Date de publication et mention de la délivrance du brevet: **17.08.2016**
(21) Numéro de dépôt: 10715955.0
(22) Date de dépôt: 18.03.2010
(51) Int. Cl.: A61K 38/17, A61K 48/00, A61P 21/00, A61K 45/06

(54) **UTILISATION DE LA DÉCORINE POUR AUGMENTER LA MASSE MUSCULAIRE**
VERWENDUNG VON DECORIN ZUR ERHÖHUNG DER MUSKLEMASSE
USE OF DECORIN FOR INCREASING MUSCLE MASS

(30) Priorité: 18.03.2009 FR 0901260
(43) Date de publication de la demande: 25.01.2012
(73) Titulaire: Association Francaise Contre les Myopathies, 75013 Paris (FR)
(72) Inventeur: KICHLER, Antoine, F-91540 Mennecy (FR); SCHERMAN, Daniel, F-75012 Paris (FR)
(74) Mandataire: Cabinet Laurent & Charras
(86) Numéro de dépôt international: PCT/FR2010/050491
(87) Numéro de publication internationale: WO 2010/106295

(56) Documents cités:
- WO-A-99/54491
- WO-A-2005/094446
- WO-A-2007/123848
- US-A1- 2005 020 491
- KISHIOKA Y ET AL: "Decorin enhances the proliferation and differentiation of myogenic cells through suppressing myostatin activity" JOURNAL OF CELLULAR PHYSIOLOGY 200806 US, vol. 215, no. 3, juin 2008 (2008-06), pages 856-867, XP002550992 ISSN: 0021-9541
- LI Y ET AL: "Decorin gene transfer promotes muscle cell differentiation and muscle regeneration" MOLECULAR THERAPY 200709 GB, vol. 15, no. 9, septembre 2007 (2007-09), pages 1616-1622, XP002550993
- ZHU JINHONG ET AL: "Relationships between transforming growth factor-beta 1, myostatin, and decorin - Implications for skeletal muscle fibrosis" JOURNAL OF BIOLOGICAL CHEMISTRY, vol. 282, no. 35, août 2007 (2007-08), pages 25852-25863, XP002550994 ISSN: 0021-9258
- MIURA T ET AL: "Decorin binds myostatin and modulates its activity to muscle cells" BIOCHEMICAL AND BIOPHYSICAL RESEARCH COMMUNICATIONS, ACADEMIC PRESS INC. ORLANDO, FL, US, vol. 340, no. 2, 10 février 2006 (2006-02-10), pages 675-680, XP024924098 ISSN: 0006-291X [extrait le 2006-02-10]
- ZANOTTI ET AL: "Altered extracellular matrix transcript expression and protein modulation in primary Duchenne muscular dystrophy myotubes" MATRIX BIOLOGY, ELSEVIER, vol. 26, no. 8, 1 octobre 2007 (2007-10-01), pages 615-624, XP022369660 ISSN: 0945-053X
- ZANOTTI S ET AL: "Decorin and biglycan expression is differentially altered in several muscular dystrophies" BRAIN 200511 GB, vol. 128, no. 11, novembre 2005 (2005-11), pages 2546-2555, XP002550995 ISSN: 0006-8950 1460-2156
- FADIC RICARDO ET AL: "Increase in decorin and biglycan in Duchenne Muscular Dystrophy: role of fibroblasts as cell source of these proteoglycans in the disease" JOURNAL OF CELLULAR AND MOLECULAR MEDICINE, vol. 10, no. 3, juillet 2006 (2006-07), pages 758-769, XP002550996 ISSN: 1582-1838
- YANG VIVIAN W -C ET AL: "Decorin is a Zn2+ metalloprotein" JOURNAL OF BIOLOGICAL CHEMISTRY, vol. 274, no. 18, 30 avril 1999 (1999-04-30), pages 12454-12460, XP002585787 ISSN: 0021-9258 cité dans la demande

### SEQUENCE LISTING

<110> GENETHON CENTRE NATIONAL DE **LA** RECHERCHE SCIENTIFIQUE
<120> UTILISATION DE **LA** DECORINE POUR AUGMENTER **LA** MASSE MUSCULAIRE
<130> G143-B-27287 PCT
<150> FR09.01260
   <151> 2009-03-18
<160> 16
<170> PatentIn version 3.3
<210> 1
   <211> 359
   <212> PRT
   <213> artificial sequence
<220>
   <223> human DCN (variant A)
<400> 1
   Met Lys Ala Thr Ile Ile Leu Leu Leu Leu Ala Gln Val Ser Trp Ala
   1 5 10 15
   Gly Pro Phe Gln Gln Arg Gly Leu Phe Asp Phe Met Leu Glu Asp Glu
   20 25 30
   Ala Ser Gly Ile Gly Pro Glu Val Pro Asp Asp Arg Asp Phe Glu Pro
   35 40 45
   Ser Leu Gly Pro Val Cys Pro Phe Arg Cys Gln Cys His Leu Arg Val
   50 55 60
   Val Gln Cys Ser Asp Leu Gly Leu Asp Lys Val Pro Lys Asp Leu Pro
   65 70 75 80
   Pro Asp Thr Thr Leu Leu Asp Leu Gln Asn Asn Lys Ile Thr Glu Ile
   85 90 95
   Lys Asp Gly Asp Phe Lys Asn Leu Lys Asn Leu His Ala Leu Ile Leu
   100 105 110
   Val Asn Asn Lys Ile Ser Lys Val Ser Pro Gly Ala Phe Thr Pro Leu
   115 120 125
   Val Lys Leu Glu Arg Leu Tyr Leu Ser Lys Asn Gln Leu Lys Glu Leu
   130 135 140
   Pro Glu Lys Met Pro Lys Thr Leu Gln Glu Leu Arg Ala His Glu Asn
   145                 150                 155                 160
   Glu Ile Thr Lys Val Arg Lys Val Thr Phe Asn Gly Leu Asn Gln Met
                   165                 170                 175
   Ile Val Ile Glu Leu Gly Thr Asn Pro Leu Lys Ser Ser Gly Ile Glu
               180                 185                 190
   Asn Gly Ala Phe Gln Gly Met Lys Lys Leu Ser Tyr Ile Arg Ile Ala
           195                 200                 205
   Asp Thr Asn Ile Thr Ser Ile Pro Gln Gly Leu Pro Pro Ser Leu Thr
       210                 215                 220
   Glu Leu His Leu Asp Gly Asn Lys Ile Ser Arg Val Asp Ala Ala Ser
   225                 230                 235                 240
   Leu Lys Gly Leu Asn Asn Leu Ala Lys Leu Gly Leu Ser Phe Asn Ser
                   245                 250                 255
   Ile Ser Ala Val Asp Asn Gly Ser Leu Ala Asn Thr Pro His Leu Arg
               260                 265                 270
   Glu Leu His Leu Asp Asn Asn Lys Leu Thr Arg Val Pro Gly Gly Leu
           275                 280                 285
   Ala Glu His Lys Tyr Ile Gln Val Val Tyr Leu His Asn Asn Asn Ile
       290                 295                 300
   Ser Val Val Gly Ser Ser Asp Phe Cys Pro Pro Gly His Asn Thr Lys
   305                 310                 315                 320
   Lys Ala Ser Tyr Ser Gly Val Ser Leu Phe Ser Asn Pro Val Gln Tyr
                   325                 330                 335
   Trp Glu Ile Gln Pro Ser Thr Phe Arg Cys Val Tyr Val Arg Ser Ala
               340                 345                 350
   Ile Gln Leu Gly Asn Tyr Lys
           355
<210> 2
   <211> 250
   <212> PRT
   <213> artificial sequence
<220>
   <223> human DCN (variant B)
<400> 2
   Met Lys Ala Thr Ile Ile Leu Leu Leu Leu Ala Gln Val Ser Trp Ala
   1               5                   10                  15
   Gly Pro Phe Gln Gln Arg Gly Leu Phe Asp Phe Met Leu Glu Asp Glu
               20                  25                  30
   Ala Ser Gly Ile Gly Pro Glu Val Pro Asp Asp Arg Asp Phe Glu Pro
           35                  40                  45
   Ser Leu Gly Pro Val Cys Pro Phe Arg Cys Gln Cys His Leu Arg Val
       50                  55                  60
   Val Gln Cys Ser Asp Leu Glu Leu Gly Thr Asn Pro Leu Lys Ser Ser
   65                  70                  75                  80
   Gly Ile Glu Asn Gly Ala Phe Gln Gly Met Lys Lys Leu Ser Tyr Ile
                   85                  90                  95
   Arg Ile Ala Asp Thr Asn Ile Thr Ser Ile Pro Gln Gly Leu Pro Pro
               100                 105                 110
   Ser Leu Thr Glu Leu His Leu Asp Gly Asn Lys Ile Ser Arg Val Asp
           115                 120                 125
   Ala Ala Ser Leu Lys Gly Leu Asn Asn Leu Ala Lys Leu Gly Leu Ser
       130                 135                 140
   Phe Asn Ser Ile Ser Ala Val Asp Asn Gly Ser Leu Ala Asn Thr Pro
   145                 150                 155                 160
   His Leu Arg Glu Leu His Leu Asp Asn Asn Lys Leu Thr Arg Val Pro
                   165                 170                 175
   Gly Gly Leu Ala Glu His Lys Tyr Ile Gln Val Val Tyr Leu His Asn
               180                 185                 190
   Asn Asn Ile Ser Val Val Gly Ser Ser Asp Phe Cys Pro Pro Gly His
           195                 200                 205
   Asn Thr Lys Lys Ala Ser Tyr Ser Gly Val Ser Leu Phe Ser Asn Pro
       210                 215                 220
   Val Gln Tyr Trp Glu Ile Gln Pro Ser Thr Phe Arg Cys Val Tyr Val
   225                 230                 235                 240
   Arg Ser Ala Ile Gln Leu Gly Asn Tyr Lys
   245 250
<210> 3
   <211> 212
   <212> PRT
   <213> artificial sequence
<220>
   <223> human DCN (variant C)
<400> 3
   Met Lys Ala Thr Ile Ile Leu Leu Leu Leu Ala Gln Val Ser Trp Ala
   1               5                   10                  15
   Gly Pro Phe Gln Gln Arg Gly Leu Phe Asp Phe Met Leu Glu Asp Glu
               20                  25                  30
   Ala Ser Gly Ile Gly Pro Glu Val Pro Asp Asp Arg Asp Phe Glu Pro
           35                  40                  45
   Ser Leu Gly Pro Val Cys Pro Phe Arg Cys Gln Cys His Leu Arg Val
       50                  55                  60
   Val Gln Cys Ser Asp Leu Gly Leu Pro Pro Ser Leu Thr Glu Leu His
   65                  70                  75                  80
   Leu Asp Gly Asn Lys Ile Ser Arg Val Asp Ala Ala Ser Leu Lys Gly
                   85                  90                  95
   Leu Asn Asn Leu Ala Lys Leu Gly Leu Ser Phe Asn Ser Ile Ser Ala
               100                 105                 110
   Val Asp Asn Gly Ser Leu Ala Asn Thr Pro His Leu Arg Glu Leu His
           115                 120                 125
   Leu Asp Asn Asn Lys Leu Thr Arg Val Pro Gly Gly Leu Ala Glu His
       130                 135                 140
   Lys Tyr Ile Gln Val Val Tyr Leu His Asn Asn Asn Ile Ser Val Val
   145                 150                 155                 160
   Gly Ser Ser Asp Phe Cys Pro Pro Gly His Asn Thr Lys Lys Ala Ser
                   165                 170                 175
   Tyr Ser Gly Val Ser Leu Phe Ser Asn Pro Val Gln Tyr Trp Glu Ile
               180                 185                 190
   Gln Pro Ser Thr Phe Arg Cys Val Tyr Val Arg Ser Ala Ile Gln Leu
           195                 200                 205
   Gly Asn Tyr Lys
       210
<210> 4
   <211> 172
   <212> PRT
   <213> artificial sequence
<220>
   <223> human DCN (variant D)
<400> 4
   Met Lys Ala Thr Ile Ile Leu Leu Leu Leu Ala Gln Val Ser Trp Ala
   1               5                   10                  15
   Gly Pro Phe Gln Gln Arg Gly Leu Phe Asp Phe Met Leu Glu Asp Glu
               20                  25                  30
   Ala Ser Gly Ile Gly Pro Glu Val Pro Asp Asp Arg Asp Phe Glu Pro
           35                  40                  45
   Ser Leu Gly Pro Val Cys Pro Phe Arg Cys Gln Cys His Leu Arg Val
       50                  55                  60
   Val Gln Cys Ser Asp Leu Gly Leu Asp Lys Val Pro Lys Asp Leu Pro
   65                  70                  75                  80
   Pro Asp Thr Thr Leu Leu Asp Leu Gln Asn Asn Lys Ile Thr Glu Ile
                   85                  90                  95
   Lys Asp Gly Asp Phe Lys Asn Leu Lys Asn Leu His Val Val Tyr Leu
               100                 105                 110
   His Asn Asn Asn Ile Ser Val Val Gly Ser Ser Asp Phe Cys Pro Pro
           115                 120                 125
   Gly His Asn Thr Lys Lys Ala Ser Tyr Ser Gly Val Ser Leu Phe Ser
       130                 135                 140
   Asn Pro Val Gln Tyr Trp Glu Ile Gln Pro Ser Thr Phe Arg Cys Val
   145                 150                 155                 160
   Tyr Val Arg Ser Ala Ile Gln Leu Gly Asn Tyr Lys
                   165                 170
<210> 5
   <211> 75
   <212> PRT
   <213> artificial sequence
<220>
   <223> human DCN (variant E)
<400> 5
   Met Lys Ala Thr Ile Ile Leu Leu Leu Leu Ala Gln Val Ser Trp Ala
   1               5                   10                  15
   Gly Pro Phe Gln Gln Arg Gly Leu Phe Asp Phe Met Leu Glu Asp Glu
               20                  25                  30
   Ala Ser Gly Ile Gly Pro Glu Val Pro Asp Asp Arg Asp Phe Glu Pro
           35                  40                  45
   Ser Leu Gly Pro Val Cys Pro Phe Arg Cys Gln Cys His Leu Arg Val
       50                  55                  60
   Val Gln Cys Ser Asp Leu Gly Cys Leu Pro Ser
   65                  70                  75
<210> 6
   <211> 354
   <212> PRT
   <213> artificial sequence
<220>
   <223> murine DCN
<400> 6
   Met Lys Ala Thr Leu Ile Phe Phe Leu Leu Ala Gln Val Ser Trp Ala
   1               5                   10                  15
   Gly Pro Phe Glu Gln Arg Gly Leu Phe Asp Phe Met Leu Glu Asp Glu
               20                  25                  30
   Ala Ser Gly Ile Ile Pro Tyr Asp Pro Asp Asn Pro Leu Ile Ser Met
           35                  40                  45
   Cys Pro Tyr Arg Cys Gln Cys His Leu Arg Val Val Gln Cys Ser Asp
       50                  55                  60
   Leu Gly Leu Asp Lys Val Pro Trp Asp Phe Pro Pro Asp Thr Thr Leu
   65                  70                  75                  80
   Leu Asp Leu Gln Asn Asn Lys Ile Thr Glu Ile Lys Glu Gly Ala Phe
                   85                  90                  95
   Lys Asn Leu Lys Asp Leu His Thr Leu Ile Leu Val Asn Asn Lys Ile
               100                 105                 110
   Ser Lys Ile Ser Pro Glu Ala Phe Lys Pro Leu Val Lys Leu Glu Arg
   115 120 125
   Leu Tyr Leu Ser Lys Asn Gln Leu Lys Glu Leu Pro Glu Lys Met Pro
   130 135 140
   Arg Thr Leu Gln Glu Leu Arg Val His Glu Asn Glu Ile Thr Lys Leu
   145 150 155 160
   Arg Lys Ser Asp Phe Asn Gly Leu Asn Asn Val Leu Val Ile Glu Leu
   165 170 175
   Gly Gly Asn Pro Leu Lys Asn Ser Gly Ile Glu Asn Gly Ala Phe Gln
   180 185 190
   Gly Leu Lys Ser Leu Ser Tyr Ile Arg Ile Ser Asp Thr Asn Ile Thr
   195 200 205
   Ala Ile Pro Gln Gly Leu Pro Thr Ser Leu Thr Glu Val His Leu Asp
   210 215 220
   Gly Asn Lys Ile Thr Lys Val Asp Ala Pro Ser Leu Lys Gly Leu Ile
   225 230 235 240
   Asn Leu Ser Lys Leu Gly Leu Ser Phe Asn Ser Ile Thr Val Met Glu
   245 250 255
   Asn Gly Ser Leu Ala Asn Val Pro His Leu Arg Glu Leu His Leu Asp
   260 265 270
   Asn Asn Lys Leu Leu Arg Val Pro Ala Gly Leu Ala Gln His Lys Tyr
   275 280 285
   Ile Gln Val Val Tyr Leu His Asn Asn Asn Ile Ser Ala Val Gly Gln
   290 295 300
   Asn Asp Phe Cys Arg Ala Gly His Pro Ser Arg Lys Ala Ser Tyr Ser
   305 310 315 320
   Ala Val Ser Leu Tyr Gly Asn Pro Val Arg Tyr Trp Glu Ile Phe Pro
   325 330 335
   Asn Thr Phe Arg Cys Val Tyr Val Arg Ser Ala Ile Gln Leu Gly Asn
   340 345 350
   Tyr Lys
<210> 7
   <211> 41
   <212> PRT
   <213> artificial sequence
<220>
   <223> murine 31-71 DCN
<400> 7
   Asp Glu Ala Ser Gly Ile Ile Pro Tyr Asp Pro Asp Asn Pro Leu Ile
   1               5                   10                  15
   Ser Met Cys Pro Tyr Arg Cys Gln Cys His Leu Arg Val Val Gln Cys
               20                  25                  30
   Ser Asp Leu Gly Leu Asp Lys Val Pro
           35                  40
<210> 8
   <211> 2305
   <212> DNA
   <213> artificial sequence
<220>
   <223> variant A1 human DCN
<400> 8
   gaatctacaa taagacaaat ttcaaatcaa gttgctccac tatactgcat aagcagttta      60
   gaatcttaag cagatgcaaa aagaataaag caaatgggag gaaaaaaaag gccgataaag     120
   tttctggcta caatacaaga gacatatcat taccatatga tctaatgtgg gtgtcagccg     180
   gattgtgttc attgagggaa accttatttt ttaactgtgc tatggagtag aagcaggagg     240
   ttttcaacct agtcacagag cagcacctac cccctcctcc tttccacacc tgcaaactct     300
   tttacttggg ctgaatattt agtgtaatta catctcagct ttgagggctc ctgtggcaaa     360
   ttcccggatt aaaaggttcc ctggttgtga aaatacatga gataaatcat gaaggccact     420
   atcatcctcc ttctgcttgc acaagtttcc tgggctggac cgtttcaaca gagaggctta     480
   tttgacttta tgctagaaga tgaggcttct gggataggcc cagaagttcc tgatgaccgc     540
   gacttcgagc cctccctagg cccagtgtgc cccttccgct gtcaatgcca tcttcgagtg     600
   gtccagtgtt ctgatttggg tctggacaaa gtgccaaagg atcttccccc tgacacaact     660
   ctgctagacc tgcaaaacaa caaaataacc gaaatcaaag atggagactt taagaacctg     720
   aagaaccttc acgcattgat tcttgtcaac aataaaatta gcaaagttag tcctggagca     780
   tttacacctt tggtgaagtt ggaacgactt tatctgtcca agaatcagct gaaggaattg     840
   ccagaaaaaa tgcccaaaac tcttcaggag ctgcgtgccc atgagaatga gatcaccaaa     900
   gtgcgaaaag ttactttcaa tggactgaac cagatgattg tcatagaact gggcaccaat 960
   ccgctgaaga gctcaggaat tgaaaatggg gctttccagg gaatgaagaa gctctcctac 1020
   atccgcattg ctgataccaa tatcaccagc attcctcaag gtcttcctcc ttcccttacg 1080
   gaattacatc ttgatggcaa caaaatcagc agagttgatg cagctagcct gaaaggactg 1140
   aataatttgg ctaagttggg attgagtttc aacagcatct ctgctgttga caatggctct 1200
   ctggccaaca cgcctcatct gagggagctt cacttggaca acaacaagct taccagagta 1260
   cctggtgggc tggcagagca taagtacatc caggttgtct accttcataa caacaatatc 1320
   tctgtagttg gatcaagtga cttctgccca cctggacaca acaccaaaaa ggcttcttat 1380
   tcgggtgtga gtcttttcag caacccggtc cagtactggg agatacagcc atccaccttc 1440
   agatgtgtct acgtgcgctc tgccattcaa ctcggaaact ataagtaatt ctcaagaaag 1500
   ccctcatttt tataacctgg caaaatcttg ttaatgtcat tgctaaaaaa taaataaaag 1560
   ctagatactg gaaacctaac tgcaatgtgg atgttttacc cacatgactt attatgcata 1620
   aagccaaatt tccagtttaa gtaattgcct acaataaaaa gaaattttgc ctgccatttt 1680
   cagaatcatc ttttgaagct ttctgttgat gttaactgag ctactagaga tattcttatt 1740
   tcactaaatg taaaatttgg agtaaatata tatgtcaata tttagtaaag cttttctttt 1800
   ttaatttcca ggaaaaaata aaaagagtat gagtcttctg taattcattg agcagttagc 1860
   tcatttgaga taaagtcaaa tgccaaacac tagctctgta ttaatcccca tcattactgg 1920
   taaagcctca tttgaatgtg tgaattcaat acaggctatg taaaattttt actaatgtca 1980
   ttattttgaa aaaataaatt taaaaataca ttcaaaatta ctattgtata caagcttaat 2040
   tgttaatatt ccctaaacac aattttatga agggagaaga cattggtttg ttgacaataa 2100
   cagtacatct tttcaagttc tcagctattt cttctacctc tccctatctt acatttgagt 2160
   atggtaactt atgtcatcta tgttgaatgt aagcttataa agcacaaagc atacatttcc 2220
   tgactggtct agagaactga tgtttcaatt tacccctctg ctaaataaat attaaaacta 2280
   tcatgtgaaa aaaaaaaaaa aaaaa 2305
<210> 9
   <211> 2151
   <212> DNA
   <213> artificial sequence
<220>
   <223> variant A2 human CN
<400> 9
   ggaataataa gacacgccct gaaggagtac atcgtctagt gagggacaga ccaagcacgc      60
   aaaacaaatt gcaatataat gtgataagtt ctttaaaaga ggtaagagca acgtgctttg     120
   ggagcagaga agagggagaa agcagcatct tgcctggatg agccagggga cacagaagag    180
   aagcccacta tctcatttaa tctttacaac tctcttgcaa ggttccctgg ttgtgaaaat    240
   acatgagata aatcatgaag gccactatca tcctccttct gcttgcacaa gtttcctggg    300
   ctggaccgtt tcaacagaga ggcttatttg actttatgct agaagatgag gcttctggga    360
   taggcccaga agttcctgat gaccgcgact tcgagccctc cctaggccca gtgtgcccct    420
   tccgctgtca atgccatctt cgagtggtcc agtgttctga tttgggtctg gacaaagtgc    480
   caaaggatct tccccctgac acaactctgc tagacctgca aaacaacaaa ataaccgaaa    540
   tcaaagatgg agactttaag aacctgaaga accttcacgc attgattctt gtcaacaata    600
   aaattagcaa agttagtcct ggagcattta cacctttggt gaagttggaa cgactttatc    660
   tgtccaagaa tcagctgaag gaattgccag aaaaaatgcc caaaactctt caggagctgc    720
   gtgcccatga gaatgagatc accaaagtgc gaaaagttac tttcaatgga ctgaaccaga    780
   tgattgtcat agaactgggc accaatccgc tgaagagctc aggaattgaa aatggggctt    840
   tccagggaat gaagaagctc tcctacatcc gcattgctga taccaatatc accagcattc    900
   ctcaaggtct tcctccttcc cttacggaat tacatcttga tggcaacaaa atcagcagag    960
   ttgatgcagc tagcctgaaa ggactgaata atttggctaa gttgggattg agtttcaaca   1020
   gcatctctgc tgttgacaat ggctctctgg ccaacacgcc tcatctgagg gagcttcact   1080
   tggacaacaa caagcttacc agagtacctg gtgggctggc agagcataag tacatccagg   1140
   ttgtctacct tcataacaac aatatctctg tagttggatc aagtgacttc tgcccacctg   1200
   gacacaacac caaaaaggct tcttattcgg gtgtgagtct tttcagcaac ccggtccagt   1260
   actgggagat acagccatcc accttcagat gtgtctacgt gcgctctgcc attcaactcg   1320
   gaaactataa gtaattctca agaaagccct catttttata acctggcaaa atcttgttaa   1380
   tgtcattgct aaaaaataaa taaaagctag atactggaaa cctaactgca atgtggatgt   1440
   tttacccaca tgacttatta tgcataaagc caaatttcca gtttaagtaa ttgcctacaa   1500
   taaaaagaaa ttttgcctgc cattttcaga atcatctttt gaagctttct gttgatgtta   1560
   actgagctac tagagatatt cttatttcac taaatgtaaa atttggagta aatatatatg   1620
   tcaatattta gtaaagcttt tcttttttaa tttccaggaa aaaataaaaa gagtatgagt   1680
   cttctgtaat tcattgagca gttagctcat ttgagataaa gtcaaatgcc aaacactagc   1740
   tctgtattaa tccccatcat tactggtaaa gcctcatttg aatgtgtgaa ttcaatacag   1800
   gctatgtaaa atttttacta atgtcattat tttgaaaaaa taaatttaaa aatacattca   1860
   aaattactat tgtatacaag cttaattgtt aatattccct aaacacaatt ttatgaaggg   1920
   agaagacatt ggtttgttga caataacagt acatcttttc aagttctcag ctatttcttc   1980
   tacctctccc tatcttacat ttgagtatgg taacttatgt catctatgtt gaatgtaagc   2040
   ttataaagca caaagcatac atttcctgac tggtctagag aactgatgtt tcaatttacc   2100
   cctctgctaa ataaatatta aaactatcat gtgaaaaaaa aaaaaaaaaa a            2151
<210> 10
   <211> 1570
   <212> DNA
   <213> artificial sequence
<220>
   <223> Variant B human DCN
<400> 10
   atgaaggcca ctatcatcct ccttctgctt gcacaagttt cctgggctgg accgtttcaa 60
   cagagaggct tatttgactt tatgctagaa gatgaggctt ctgggatagg cccagaagtt 120
   cctgatgacc gcgacttcga gccctcccta ggcccagtgt gccccttccg ctgtcaatgc 180
   catcttcgag tggtccagtg ttctgatttg gaactgggca ccaatccgct gaagagctca 240
   ggaattgaaa atggggcttt ccagggaatg aagaagctct cctacatccg cattgctgat 300
   accaatatca ccagcattcc tcaaggtctt cctccttccc ttacggaatt acatcttgat 360
   ggcaacaaaa tcagcagagt tgatgcagct agcctgaaag gactgaataa tttggctaag 420
   ttgggattga gtttcaacag catctctgct gttgacaatg gctctctggc caacacgcct 480
   catctgaggg agcttcactt ggacaacaac aagcttacca gagtacctgg tgggctggca 540
   gagcataagt acatccaggt tgtctacctt cataacaaca atatctctgt agttggatca 600
   agtgacttct gcccacctgg acacaacacc aaaaaggctt cttattcggg tgtgagtctt 660
   ttcagcaacc cggtccagta ctgggagata cagccatcca ccttcagatg tgtctacgtg 720
   cgctctgcca ttcaactcgg aaactataag taattctcaa gaaagccctc atttttataa 780
   cctggcaaaa tcttgttaat gtcattgcta aaaaataaat aaaagctaga tactggaaac 840
   ctaactgcaa tgtggatgtt ttacccacat gacttattat gcataaagcc aaatttccag 900
   tttaagtaat tgcctacaat aaaaagaaat tttgcctgcc attttcagaa tcatcttttg 960
   aagctttctg ttgatgttaa ctgagctact agagatattc ttatttcact aaatgtaaaa 1020
   tttggagtaa atatatatgt caatatttag taaagctttt cttttttaat ttccaggaaa 1080
   aaataaaaag agtatgagtc ttctgtaatt cattgagcag ttagctcatt tgagataaag 1140
   tcaaatgcca aacactagct ctgtattaat ccccatcatt actggtaaag cctcatttga 1200
   atgtgtgaat tcaatacagg ctatgtaaaa tttttactaa tgtcattatt ttgaaaaaat 1260
   aaatttaaaa atacattcaa aattactatt gtatacaagc ttaattgtta atattcccta 1320
   aacacaattt tatgaaggga gaagacattg gtttgttgac aataacagta catcttttca 1380
   agttctcagc tatttcttct acctctccct atcttacatt tgagtatggt aacttatgtc 1440
   atctatgttg aatgtaagct tataaagcac aaagcataca tttcctgact ggtctagaga 1500
   actgatgttt caatttaccc ctctgctaaa taaatattaa aactatcatg tgaaaaaaaa 1560
   aaaaaaaaaa 1570
<210> 11
   <211> 1456
   <212> DNA
   <213> artificial sequence
<220>
   <223> variant C human DCN
<400> 11
   atgaaggcca ctatcatcct ccttctgctt gcacaagttt cctgggctgg accgtttcaa      60
   cagagaggct tatttgactt tatgctagaa gatgaggctt ctgggatagg cccagaagtt     120
   cctgatgacc gcgacttcga gccctcccta ggcccagtgt gccccttccg ctgtcaatgc     180
   catcttcgag tggtccagtg ttctgatttg ggtcttcctc cttcccttac ggaattacat     240
   cttgatggca acaaaatcag cagagttgat gcagctagcc tgaaaggact gaataatttg     300
   gctaagttgg gattgagttt caacagcatc tctgctgttg acaatggctc tctggccaac     360
   acgcctcatc tgagggagct tcacttggac aacaacaagc ttaccagagt acctggtggg     420
   ctggcagagc ataagtacat ccaggttgtc taccttcata acaacaatat ctctgtagtt     480
   ggatcaagtg acttctgccc acctggacac aacaccaaaa aggcttctta ttcgggtgtg     540
   agtcttttca gcaacccggt ccagtactgg gagatacagc catccacctt cagatgtgtc     600
   tacgtgcgct ctgccattca actcggaaac tataagtaat tctcaagaaa gccctcattt     660
   ttataacctg gcaaaatctt gttaatgtca ttgctaaaaa ataaataaaa gctagatact     720
   ggaaacctaa ctgcaatgtg gatgttttac ccacatgact tattatgcat aaagccaaat     780
   ttccagttta agtaattgcc tacaataaaa agaaattttg cctgccattt tcagaatcat     840
   cttttgaagc tttctgttga tgttaactga gctactagag atattcttat ttcactaaat     900
   gtaaaatttg gagtaaatat atatgtcaat atttagtaaa gcttttcttt tttaatttcc     960
   aggaaaaaat aaaaagagta tgagtcttct gtaattcatt gagcagttag ctcatttgag    1020
   ataaagtcaa atgccaaaca ctagctctgt attaatcccc atcattactg gtaaagcctc    1080
   atttgaatgt gtgaattcaa tacaggctat gtaaaatttt tactaatgtc attattttga    1140
   aaaaataaat ttaaaaatac attcaaaatt actattgtat acaagcttaa ttgttaatat    1200
   tccctaaaca caattttatg aagggagaag acattggttt gttgacaata acagtacatc    1260
   ttttcaagtt ctcagctatt tcttctacct ctccctatct tacatttgag tatggtaact    1320
   tatgtcatct atgttgaatg taagcttata aagcacaaag catacatttc ctgactggtc    1380
   tagagaactg atgtttcaat ttacccctct gctaaataaa tattaaaact atcatgtgaa    1440
   aaaaaaaaaa aaaaaa                                                    1456
<210> 12
   <211> 1336
   <212> DNA
   <213> Artificial sequence
<220>
   <223> variant D human DCN
<400> 12
   atgaaggcca ctatcatcct ccttctgctt gcacaagttt cctgggctgg accgtttcaa      60
   cagagaggct tatttgactt tatgctagaa gatgaggctt ctgggatagg cccagaagtt     120
   cctgatgacc gcgacttcga gccctcccta ggcccagtgt gccccttccg ctgtcaatgc     180
   catcttcgag tggtccagtg ttctgatttg ggtctggaca aagtgccaaa ggatcttccc     240
   cctgacacaa ctctgctaga cctgcaaaac aacaaaataa ccgaaatcaa agatggagac     300
   tttaagaacc tgaagaacct tcacgttgtc taccttcata acaacaatat ctctgtagtt     360
   ggatcaagtg acttctgccc acctggacac aacaccaaaa aggcttctta ttcgggtgtg     420
   agtcttttca gcaacccggt ccagtactgg gagatacagc catccacctt cagatgtgtc     480
   tacgtgcgct ctgccattca actcggaaac tataagtaat tctcaagaaa gccctcattt     540
   ttataacctg gcaaaatctt gttaatgtca ttgctaaaaa ataaataaaa gctagatact     600
   ggaaacctaa ctgcaatgtg gatgttttac ccacatgact tattatgcat aaagccaaat     660
   ttccagttta agtaattgcc tacaataaaa agaaattttg cctgccattt tcagaatcat     720
   cttttgaagc tttctgttga tgttaactga gctactagag atattcttat ttcactaaat     780
   gtaaaatttg gagtaaatat atatgtcaat atttagtaaa gcttttcttt tttaatttcc     840
   aggaaaaaat aaaaagagta tgagtcttct gtaattcatt gagcagttag ctcatttgag     900
   ataaagtcaa atgccaaaca ctagctctgt attaatcccc atcattactg gtaaagcctc     960
   atttgaatgt gtgaattcaa tacaggctat gtaaaatttt tactaatgtc attattttga    1020
   aaaaataaat ttaaaaatac attcaaaatt actattgtat acaagcttaa ttgttaatat    1080
   tccctaaaca caattttatg aagggagaag acattggttt gttgacaata acagtacatc    1140
   ttttcaagtt ctcagctatt tcttctacct ctccctatct tacatttgag tatggtaact    1200
   tatgtcatct atgttgaatg taagcttata aagcacaaag catacatttc ctgactggtc    1260
   tagagaactg atgtttcaat ttacccctct gctaaataaa tattaaaact atcatgtgaa    1320
   aaaaaaaaaa aaaaaa                                                    1336
<210> 13
   <211> 1223
   <212> DNA
   <213> Artificial sequence
<220>
   <223> Variant E human DCN
<400> 13
   atgaaggcca ctatcatcct ccttctgctt gcacaagttt cctgggctgg accgtttcaa      60
   cagagaggct tatttgactt tatgctagaa gatgaggctt ctgggatagg cccagaagtt     120
   cctgatgacc gcgacttcga gccctcccta ggcccagtgt gccccttccg ctgtcaatgc     180
   catcttcgag tggtccagtg ttctgatttg ggttgtctac cttcataaca acaatatctc     240
   tgtagttgga tcaagtgact tctgcccacc tggacacaac accaaaaagg cttcttattc     300
   gggtgtgagt cttttcagca acccggtcca gtactgggag atacagccat ccaccttcag     360
   atgtgtctac gtgcgctctg ccattcaact cggaaactat aagtaattct caagaaagcc     420
   ctcattttta taacctggca aaatcttgtt aatgtcattg ctaaaaaata aataaaagct     480
   agatactgga aacctaactg caatgtggat gttttaccca catgacttat tatgcataaa     540
   gccaaatttc cagtttaagt aattgcctac aataaaaaga aattttgcct gccattttca     600
   gaatcatctt ttgaagcttt ctgttgatgt taactgagct actagagata ttcttatttc     660
   actaaatgta aaatttggag taaatatata tgtcaatatt tagtaaagct tttctttttt     720
   aatttccagg aaaaaataaa aagagtatga gtcttctgta attcattgag cagttagctc     780
   atttgagata aagtcaaatg ccaaacacta gctctgtatt aatccccatc attactggta     840
   aagcctcatt tgaatgtgtg aattcaatac aggctatgta aaatttttac taatgtcatt     900
   attttgaaaa aataaattta aaaatacatt caaaattact attgtataca agcttaattg     960
   ttaatattcc ctaaacacaa ttttatgaag ggagaagaca ttggtttgtt gacaataaca    1020
   gtacatcttt tcaagttctc agctatttct tctacctctc cctatcttac atttgagtat    1080
   ggtaacttat gtcatctatg ttgaatgtaa gcttataaag cacaaagcat acatttcctg    1140
   actggtctag agaactgatg tttcaattta cccctctgct aaataaatat taaaactatc    1200
   atgtgaaaaa aaaaaaaaaa aaa                                             1223
<210> 14
   <211> 1065
   <212> DNA
   <213> Artificial sequence
<220>
   <223> Murine DCN
<400> 14
   atgaaggcaa ctctcatctt cttccttctg gcacaagtct cttgggctgg accatttgaa      60
   cagagaggct tatttgactt catgctagaa gatgaggctt ctggcataat cccttatgac     120
   cctgacaatc ccctgatatc tatgtgcccc taccgatgcc agtgtcatct tcgagtggtg     180
   cagtgttctg atctgggttt ggacaaagtg ccctgggatt ttccacccga cacaaccttg    240
   ctagacctgc aaaacaacaa aattacagag atcaaagaag gggccttcaa gaacctgaag    300
   gacttgcata ccttgatcct tgtcaacaac aagatcagca aaatcagtcc agaggcattc    360
   aaacctctcg tgaagttgga aaggctttac ctgtctaaga accaactaaa ggaactgcct    420
   gaaaaaatgc ccagaactct ccaggaactt cgtgtccatg agaatgagat caccaagctg    480
   cggaaatccg acttcaatgg actgaacaat gtgcttgtca tagaactggg cggcaaccca    540
   ctgaaaaact ctgggattga aaacggagcc ttccagggac tgaagagtct ctcatacatt    600
   cgcatctcag acaccaacat aactgcgatc cctcaaggtc tgcctacttc tctcactgaa    660
   gtgcatctag atggcaacaa gatcaccaag gttgatgcac ccagcctgaa aggactgatt    720
   aatttgtcta aactgggatt gagcttcaac agcatcaccg ttatggagaa tggcagtctg    780
   gccaatgttc ctcatctgag ggaactccac ttggacaaca acaaactcct cagggtgcct    840
   gctgggctgg cacagcataa gtatatccag gtcgtctacc ttcacaacaa caacatctcc    900
   gcagttgggc aaaatgactt ctgccgagct ggacacccct ctcgaaaggc ttcctactcg    960
   gctgtgagtc tttacggcaa ccctgtccgg tattgggaaa tctttccaaa caccttcaga   1020
   tgtgtctatg tgcgttctgc cattcaactt ggaaactaca agtaa                   1065
<210> 15
   <211> 30
   <212> PRT
   <213> artificial sequence
<220>
   <223> murine 42-71 DCN
<400> 15
   Asp Asn Pro Leu Ile Ser Met Cys Pro Tyr Arg Cys Gln Cys His Leu
   1               5                   10                  15
   Arg Val Val Gln Cys Ser Asp Leu Gly Leu Asp Lys Val Pro
               20                  25                  30
<210> 16
   <211> 41
   <212> PRT
   <213> artificial sequence
<220>
   <223> human DCN fragment
<400> 16
   Ile Gly Pro Glu Val Pro Asp Asp Arg Asp Phe Glu Pro Ser Leu Gly
   1               5                   10                  15
   Pro Val Cys Pro Phe Arg Cys Gln Cys His Leu Arg Val Val Gln Cys
               20                  25                  30
   Ser Asp Leu Gly Leu Asp Lys Val Pro
           35                  40

## Description

### DOMAINE TECHNIQUE

La présente invention vise à augmenter la masse musculaire chez l'homme ou l'animal.

Plus précisément, elle préconise l'utilisation d'un fragment de la décorine apte à fixer le zinc de moins de 100 acides aminés pour développer la masse musculaire, et notamment traiter des conditions pathologiques associées à une fonte musculaire, telles que des dystrophies musculaires.

### ETAT ANTERIEUR DE LA TECHNIQUE

Les maladies neuromusculaires regroupent des pathologies diverses qui sont généralement associées à une perte de force musculaire transitoire ou permanente. Cette perte de force s'accompagne le plus souvent d'une fonte musculaire, également appelée amyotrophie.

Parmi ces maladies musculaires, les myopathies constituent un groupe important correspondant à des atteintes de la fibre musculaire à proprement parler. Parmi elles, les dystrophies musculaires progressives se caractérisent par une diminution de la force musculaire avec généralement une atrophie des muscles, ainsi que par des anomalies à la biopsie musculaire révélant une modification du tissu. Appartiennent notamment à ce groupe la dystrophie musculaire de Duchenne (ou DMD), la dystrophie musculaire de Becker (ou DMB), ainsi que les myopathies des ceintures.

Pour certaines de ces pathologies, les anomalies génétiques associées ont pu être identifiées. Ainsi, les dystrophies musculaires de Duchenne ou de Becker sont liées à des altérations dans le gène codant la dystrophine, la dystrophie des ceintures de type 2A (LGMD 2A ou calpainopathie) à des altérations dans le gène de la calpaïne 3, les sarcoglycanopathies ou myopathies des ceintures de type LGMD 2C, LGMD 2D, LGMD 2E, LGMD 2F à des défauts dans les gènes des γ-, α-, β- et δ-sarcoglycanes, respectivement (McNally EM, Pytel P, Muscle diseases : the muscular dystrophies. Annu Rev Pathol. 2007 ; Vol 2 :87-109).

Dans ces cas particuliers, différentes stratégies de thérapie génique sont en cours de développement mais restent délicates à mettre en oeuvre.

Toutefois et plus généralement dans tous les cas de fonte musculaire, il existe un besoin évident de développer des solutions techniques permettant d'augmenter la masse et/ou le volume musculaire.

Ainsi, le document WO 2005/094446 a identifié des anticorps dirigés contre un épitope localisé entre les résidus 40 et 64 de la myostatine humaine mature susceptibles d'augmenter la masse musculaire. Toutefois, cette stratégie basée sur la reconnaissance de la myostatine par un anticorps n'est pas sans poser de problèmes. Il existe donc le besoin de trouver des solutions alternatives.

La présente invention repose sur la mise en évidence, par les inventeurs, de cette propriété chez certains fragments de la décorine.

La décorine appartient à la famille des protéines SLRP (Small Leucine-Rich Proteoglycan) et comprend un segment LRR (Leucine-Rich Repeat). La décorine fait partie de la classe I des SLRP. Les membres de cette famille sont sécrétés avec un propeptide qui, dans certains cas, est clivé. La décorine présente également une chaîne glycosaminoglycane (GAG).

La décorine est une protéine de la matrice extracellulaire, de structure proche de la protéine biglycan. Elle joue un rôle dans l'assemblage de la matrice et interagit avec différents partenaires, tels que le collagène de type I, II, III et IV, ou encore le facteur TGF-beta (Ameye L, Young MF, Mice deficient in small leucine-rich proteoglycans : novel in vivo models for osteoporosis, osteoarthritis, Ehlers Danlos syndrome, muscular dystrophy, and corneal diseases. Glycobiology 2002 ; Vol 12 :107R-116R ; Reed CC, Iozzo RV, The role of decorin in collagen fibrillogenesis and skin homeostasis. Glycoconj J. 2002 ; Vol 19(4-5) :249-55).

Ainsi et sur la base de son interaction avec le facteur TGF-beta, le document WO 96/25178 a proposé d'utiliser la décorine pour traiter les pathologies associées à la fibrose tissulaire, c'est-à-dire à une production excessive de matrice extracellulaire, sans rapport avec la problématique de la masse musculaire.
Il a aussi été mentionné, dans WO 2007/123848 et WO 99/54491, la possibilité d'utiliser la décorine entière pour le traitement de maladies musculaires. Par ailleurs, les documents Kishioka et al. (Journal of cellular physiology, 2008, 215(3):856-67), Li et al. (Molecular therapy, 2007, 15(9):1616-22), Zhu et al. (Journal of biological chemistry, 2007, 282(35):25852-63), Miura et al. (Biochemical and biophysical research communications, 2006, 340(2):675-80) et Zanotti et al. (Matrix biology, 2007, 26(8):615-24) ont rapporté des expériences menées *in vitro* tendant à démontrer un effet anti-myostatine de la décorine entière ou son effet sur la fibrose, la prolifération et la différentiation musculaire. Il était également connu de Miura *et al.* que l'interaction entre la décorine et la myostatine est zinc dépendante, des fragments de la décorine aptes à fixer le zinc ayant par ailleurs été décrits dans les documents Yang et al. (Journal of biological chemistry, 1999, 274(18): 12454-60) et US 2005/020491.

### DESCRIPTION DETAILLEE DE L'INVENTION

La présente invention concerne donc l'utilisation de certains fragments de la décorine pour lutter contre la fonte musculaire, voire pour augmenter la masse musculaire.

Dans le cadre de l'invention, les termes « masse musculaire » pourraient indifféremment être remplacés par poids ou volume musculaire.

Ainsi et de manière plus précise, l'invention vise aussi bien :
- une composition comprenant un fragment de la décorine apte à fixer le zinc de moins de 100 acides aminés pour traiter des pathologies associées à une fonte musculaire ;
- l'utilisation d'un fragment de la décorine apte à fixer le zinc de moins de 100 acides aminés pour la préparation d'un médicament destiné à traiter des pathologies associées à une fonte musculaire ;
- l'utilisation non thérapeutique d'une composition comprenant un fragment de la décorine apte à fixer le zinc de moins de 100 acides aminés pour augmenter la masse musculaire.

En effet, il existe un certain nombre de conditions dans lesquelles la fonte musculaire se produit.

Il peut tout d'abord s'agir de conditions pathologiques, en particulier dans le cas des maladies neuromusculaires. Ainsi, la myopathie de Duchenne est une pathologie particulièrement visée mais toutes les formes de maladies neuromusculaires, en particulier les dystrophies musculaires, peuvent être traitées.

En outre, la cachexie ou marasme est également un état pathologique visé par la présente invention. Cet état se caractérise par une maigreur extrême, en particulier au niveau musculaire, provoquée par une longue maladie ou un apport protéinique ou calorique insuffisant.

Cet état pathologique est notamment observé dans les cas de maladies chroniques telles que le cancer ou le SIDA ou chez les personnes souffrant soit d'insuffisance cardiaque - il y a une atrophie des muscles squelettiques chez 68% des patients -, soit d'incontinence urinaire.

Sans être considérées comme pathologiques à proprement parler, certaines situations sont associées à une fonte musculaire : vieillissement, immobilisation prolongée.... Il existe donc là encore un intérêt à augmenter la masse musculaire.

En outre et notamment dans le domaine agro-alimentaire, l'invention offre la possibilité d'augmenter la production de viande animale. L'utilisation de certains fragments de la décorine présente donc un intérêt tout particulier chez l'animal.

Ainsi, la présente invention repose sur la mise en évidence de propriétés stimulantes de fragments de la décorine sur le volume musculaire notamment.

Dans le cadre de l'invention, on appelle, de manière générique, « décorine » la protéine décrite par Krusius *et al.* (Krusius T., Ruoslahti E., Primary structure of an extracellular matrix proteoglycan core protein deduced from cloned cDNA. Proc Natl Acad Sci USA 1986 ; Vol 83(20):7683-87). La protéine humaine décrite dans ce document présente la séquence SEQ ID NO : 1. Elle se présente sous la forme d'une préproprotéine de 359 acides aminés. Aussi bien les protéines natives, que celles dépourvues de leur propeptide et/ou de leur séquence signal (329 aa), sont visées par la présente invention.

Même si la décorine à l'état naturel présente une chaîne glycosaminoglycane (GAG), une décorine dépourvue de GAG (GAG-) peut également être mise en oeuvre dans le cadre de l'invention. Celle-ci peut par exemple être obtenue par traitement enzymatique.

La décorine peut provenir de tout organisme mais, dans le cadre de l'invention, la décorine d'origine humaine est privilégiée. Plus généralement et de manière avantageuse, la protéine provient du même organisme que celui dans lequel elle va être administrée. Ainsi et de manière privilégiée, pour les indications thérapeutiques chez l'homme, une décorine humaine sera avantageusement utilisée.

En effet, un des avantages primordiaux de la solution proposée dans le cadre de la présente invention est que la décorine est une protéine naturellement présente chez les mammifères, en particulier l'homme, et donc *a priori* non susceptibles d'entraîner des effets secondaires ou des réponses immunes.

Concernant la décorine humaine, il a également été montré qu'il existait des variants transcriptionnels (variants b, c, d et e), donnant lieu à des protéines isoformes de séquence SEQ ID NO : 2, 3, 4 et 5, respectivement, décrites dans la présente demande.

Il apparaît donc que dans le cadre de l'invention, le terme « décorine » a un sens large et décrit aussi bien :
- la protéine native, notamment de séquence SEQ ID NO : 1;
- la protéine avec ou sans la chaîne GAG (GAG+ ou GAG-, respectivement) ;
- la protéine dépourvue du propeptide et/ou de la séquence signal ;
- des variants de ces protéines, notamment incarnés par les séquences SEQ ID NOS : 2 à 5.

Concernant les fragments selon l'invention ou les dérivés décrits dans la présente demande, il s'agit bien sûr de fragments ou dérivés actifs. L'activité visée que doivent posséder ces fragments ou dérivés concerne la capacité à augmenter la masse musculaire, laquelle est aisément évaluée grâce à la mise en oeuvre du test décrit dans la présente demande.

En pratique, ceux-ci présentent avantageusement 60% d'identité avec l'une des séquences SEQ ID NO : 1 à 7, encore plus avantageusement 70%, 80%, voire 90% d'identité.

Ainsi, la demande décrit aussi, à titre d'exemple pour les dérivés, la séquence SEQ ID NO : 6 correspondant à la protéine murine de 354 aa, qui présente 80% d'identité avec la séquence humaine SEQ ID NO : 1.

Selon un mode de réalisation privilégié de l'invention, il s'agit d'un fragment actif de la décorine. On entend par « fragment » selon l'invention, un peptide comportant moins de 100 acides aminés, avantageusement moins de 50 acides aminés.

La mise en oeuvre d'un peptide en lieu et place de la protéine présente des avantages certains, notamment au niveau de sa production mais également concernant le risque éventuel d'interférences indésirables *in vivo.*

Or, il a été démontré dans le cadre de la présente demande qu'un fragment de 41 résidus de la partie N-terminale de la décorine murine (SEQ ID NO : 7) correspondant aux résidus 31 à 71 de la séquence SEQ ID NO : 6, rapporté pour fixer le zinc (Yang VW, LaBrenz SR, Rosenberg LC, McQuillan D, Höök M. Decorin is a Zn2+ metalloprotein. J Biol Chem. 1999, 274(18):12454-60), présentait l'activité recherchée. Il a également été démontré qu'un fragment encore plus petit de 30 résidus (résidus 42 à 71 de la séquence SEQ ID NO : 6 correspondant à SEQ ID NO : 15) était également actif.

Le domaine correspondant, présent dans la décorine humaine, peut être aisément déterminé grâce à la méthodologie décrite dans ce document. Un tel fragment peut par exemple présenter la séquence SEQ ID NO : 16.

De manière plus générale, l'invention concerne donc la mise en oeuvre d'un fragment de la décorine comprenant le domaine de fixation au zinc, en pratique les résidus 31 à 71, éventuellement 42 à 71 de la séquence murine. Dans un mode de réalisation particulier, le fragment en question présente une séquence correspondant à la séquence SEQ ID NO: 7 ou SEQ ID NO: 15. En outre, des fragments présentant avantageusement 50% d'identité avec la séquence SEQ ID NO : 7 ou 15, encore plus avantageusement 60%, 70%, 80%, voire 90% d'identité, et conservant leur capacité à fixer le zinc sont également visés par la présente invention.

En outre, les fragments selon l'invention et les dérivés actifs décrits dans la demande peuvent également se présenter sous forme de protéine fusion ou protéine chimérique, avec un autre fragment protéique à leur extrémité N ou C-terminale. Celles-ci peuvent par exemple, mais de façon non limitative, augmenter le temps de résidence de la protéine dans l'organisme. Un exemple privilégié est la chimère constituée de la région constante des IgG de mammifère, associée via une séquence charnière à la décorine ou l'un de ses fragments. Un autre exemple est l'albumine humaine ou de mammifère, associée elle-aussi à la décorine ou à un fragment protéique issu de la décorine. De telles associations peuvent aussi bien être obtenues à partir d'un ADNc recombiné que par liaison chimique des 2 protéines.

La présente invention repose donc sur un apport exogène d'un fragment de décorine. De fait, la composition visée par l'invention comprend soit le fragment en tant que tel, soit un système de production du fragment.

Concernant le fragment selon l'invention, il peut aussi bien s'agir d'un fragment de la décorine native, purifiée à partir d'un organisme produisant naturellement cette protéine, ou bien d'un fragment d'une protéine recombinante produite à partir d'un quelconque des systèmes de synthèse disponibles et connus de l'homme du métier.

Alternativement, une séquence d'acides nucléiques codant pour le fragment selon l'invention est placée dans un système d'expression, avantageusement sous le contrôle d'un promoteur dans un vecteur. Après introduction dans l'organisme, le fragment est produit *in vivo.* Le transfert des acides nucléiques (ADN ou ARN) peut se faire soit avec des approches virales de transfert de gènes (par exemple virus adéno-associé ou AAV), soit avec des approches non virales (par exemple par simple injection intramusculaire d'un plasmide). Un ADN génomique peut avoir un intérêt puisque dans certains cas, la présence des introns stabilise l'ARNm préépissé et améliore sa stabilité dans le noyau et son exportation, ce qui conduit à une meilleure expression protéique.

Ainsi, les fragments selon l'invention ou les dérivés décrits dans la présente demande peuvent être fournis sous forme d'acides nucléiques, notamment ADN ou ARN. Il peut par exemple s'agir de fragments ou dérivés issus des transcrits naturellement présents chez l'homme ou la souris. Ainsi, les séquences des transcrits suivants sont décrites :
- La séquence SEQ ID NO : 8 correspond au variant A1 (Accession Number NM_001920.3), qui est le plus long des transcrit et code pour l'isoforme a de la décorine humaine de séquence SEQ ID NO: 1 (Accession Number NP_001911);
- La séquence SEQ ID NO : 9 correspond au variant A2 (Accession Number NM_133503.2), qui utilise un exon alternatif au niveau du 5'UTR en comparaison avec le variant A1 et code pour la même protéine de séquence SEQ ID NO : 1 (Accession Number NP_598010.1) ;
- La séquence SEQ ID NO : 10 correspond au variant B (Accession Number NM_133504.2), qui est dépourvu des exons 3 et 4 dans la région codante, en comparaison avec le variant A1. Ceci ne cause pas de changement de phase de lecture mais code pour une isoforme b de la protéine, dépourvue d'un fragment interne de 109 aa et de séquence SEQ ID NO: 2 (Accession Number NP_598011.1);
- La séquence SEQ ID NO : 11 correspond au variant C (Accession Number NM_133505.2), qui est dépourvu des exons 3, 4 et 5 dans la région codante, en comparaison avec le variant A1. Ceci cause un changement de phase de lecture
- interne et l'isoforme c codée, de séquence SEQ ID NO : 3 (Accession Number NP_598012.1) est plus courte de 147 acides aminés par rapport à l'isoforme a ;
- La séquence SEQ ID NO : 12 correspond au variant D (Accession Number NM_133506.2), qui est dépourvu des exons 4, 5, 6 et 7 dans la région codante, en comparaison avec le variant A1. Ceci ne cause pas de changement de phase de lecture mais code pour une isoforme d de la protéine, dépourvue d'un fragment interne de 187 aa, de séquence SEQ ID NO : 4 (Accession Number NP_598013.1);
- La séquence SEQ ID NO : 13 correspond au variant E (Accession Number NM_133507.2), qui est dépourvu des exons 3, 4, 5, 6 et 7 dans la région codante, en comparaison avec le variant A1. Ceci cause un changement de phase de lecture et l'isoforme e codée, de séquence SEQ ID NO : 5 (Accession Number NP_598014.1) est plus courte de 284 acides aminés par rapport à l'isoforme a ;
- La séquence SEQ ID NO : 14 code pour la protéine murine de séquence SEQ ID NO : 6 (Accession Number P28654).

Comme déjà dit, la décorine est connue pour être une métalloprotéine à zinc. De ce fait et dans le but de potentialiser son activité, il peut être choisi d'apporter du zinc, en plus de celui naturellement disponible dans l'organisme auquel est administrée le fragment de décorine. Ainsi et selon ce mode de réalisation, la composition comprenant le fragment selon l'invention comprend également du zinc, par exemple sous forme de chlorure de zinc, avantageusement à une concentration comprise entre 1 et 50 µM, voire égale à 15 µM.

Une composition comprenant le fragment de décorine selon l'invention, destinée au traitement des pathologies associées à une fonte musculaire, peut en outre contenir tout composé ou excipient acceptable, notamment pharmaceutique. La voie d'administration peut aussi bien être intramusculaire qu'intraveineuse, voire sous-cutanée, intrapéritonéale ou orale.

Pour favoriser la prise de greffe de cellules précurseurs ou cellules souches, il peut être avantageux d'associer l'administration du fragment selon l'invention à la greffe de cellules (myoblastes, cellules souches, ....). Cette administration peut se faire de manière simultanée ou décalée dans le temps.

Il peut aussi être avantageux de combiner une thérapie génique, destinée au traitement d'une maladie neuromusculaire, avec l'administration de la décorine. Ainsi et selon un mode de réalisation privilégié, un gène thérapeutique est associé au traitement par le fragment de décorine. L'administration des 2 traitements peut se faire de manière simultanée ou décalée dans le temps.

Les effets avantageux du fragment selon l'invention se traduisent par une augmentation du volume (ou masse ou poids) musculaire, due à une augmentation de la surface des fibres associée éventuellement à une augmentation du nombre de fibres. Ces effets positifs peuvent être observés pour les différents muscles squelettiques, aussi bien chez un organisme atteint d'une pathologie affectant sa masse musculaire que chez un individu sain. A priori, aucun effet secondaire, ni aucune réaction immunologique n'est à déplorer.

### EXEMPLES DE REALISATION

L'invention et les avantages qui en découlent ressortiront mieux des exemples de réalisation suivants, à l'appui des figures annexées. Ceux-ci n'ont toutefois aucune portée limitative.

L'invention est illustrée plus avant à l'aide d'une décorine recombinante de souris, injectée par voie intramusculaire dans des souris *mdx* possédant un gène codant une dystrophine altérée et servant de modèle d'étude pour la myopathie de Duchenne, ainsi que des souris gamma-sarcoglycan -/- (modèle murin de sarcoglycanopathies sur fond pur C57/B16).

### LEGENDES DES FIGURES

La figure 1 est une vue du muscle *tibialis antérieur* prélevé sur la souris *mdx* 7, ayant reçu (à gauche) ou non (à droite) une injection intramusculaire de décorine.
La figure 2 est une vue du muscle *tibialis antérieur* prélevé sur la souris *mdx* 8, ayant reçu (à gauche) ou non (à droite) une injection intramusculaire de décorine.
La figure 3 est une vue du muscle *tibialis antérieur* prélevé sur la souris *gamma-sarco* -/- 4 à J18, ayant reçu (à gauche) ou non (à droite) une injection intramusculaire de décorine.
La figure 4 est une vue d'une coupe transversale du muscle *tibialis antérieur* prélevé sur la souris *gamma-sarco* -/- 4 à J18, ayant reçu (TAG4 à droite de la figure) ou non (TAD4 à gauche de la figure) une injection intramusculaire de décorine.

### I) MATERIELS ET METHODES

### Réparation de la solutions de mDécorine

La protéine utilisée est de la décorine recombinante de souris (mDécorine) de séquence SEQ ID NO : 6, fournie par la société R&D systems.

24-40 heures avant l'injection, 100 µl de NaCl stérile à 150 mM et 6 µl de ZnCl₂ à 250 µM sont ajoutés à 100 µg de mDécorine en poudre. Le volume final est de 106 µl avec une concentration finale d'environ 1 µg/µl. Pour les injections dans les muscles contrôles, 100 µl de NaCl à 150 mM et 6 µl de ZnCl₂ à 250 µM sont également mélangés. Toutes ces solutions, après avoir été vortexées, sont conservées à 4°C.

### Injection in vivo

Toutes les souris sont traitées selon les directives Européennes pour la santé humaine et l'utilisation des animaux expérimentaux.

Des souris dystrophiques *mdx* (S-linked muscular dystrophy) ou gamma-sarcoglycan - /-, âgées d'au moins 6 semaines, ont été utilisées. Dans le *tibialis intérieur gauche* (TAG) qui correspond au muscle traité, 20 µg mDécorine -soit 22 µl de la solution décrite ci-dessus (20 µg Décorine + 15 µM ZnCl₂ / 22 µl NaCl)- ont été injectés. Pour le muscle témoin *tibialis intérieur droit* (TAD), 22 µl de la solution témoin décrite ci-dessus (15 µM ZnCl₂ / 22 µl NaCl) ont été administrés. Après un nombre déterminé de jours après l'injection, les TAD et TAG sont prélevés après sacrifice des souris, pesés puis congelés en vue d'études histologiques.

### Préparation et injection de la solutions contenant le Peptide mDCN 31-71 :

Le peptide ayant la séquence SEQ ID NO : 7 a été synthétisé par la société NeoMPS avec une pureté > 65%. Il a été dissout à 2 mg/mL dans du NaCl 150 mM et stocké à - 80°C.

Pour les injections, le protocole de préparation est identique à celui utilisé pour la protéine, à savoir : 24-40 heures avant l'injection, la quantité désirée de peptide est prélevée de la solution mère et mélangée avec une solution de chlorure de zinc (ZnCl₂) et de NaCl 150 mM, de manière à avoir une concentration finale de 15 µM en zinc. Le protocole d'injection est identique à celui utilisée pour la protéine.

### Analyses histologiques

### - Marquage laminine :

Des sections cryostat (8 µm) des muscles traités et témoins sont réalisés suivant les techniques standards. Les lames sont fixées avec du Dakopen (DAKO^{®}, Réf. : S 2002) pendant 10 minutes à l'air libre et ensuite bloquées avec une solution de PBS / sérum de chèvre 10%, pendant 30 min à température ambiante et en chambre humide. L'anticorps lapin anti laminine (DAKO^{®}, Réf. : Z0097) est appliqué sur les lames à une dilution de 1 : 1000 pendant 12h en chambre humide. Les lames sont alors rincées dans du PSB (5 minutes) sous agitation et l'anticorps secondaire (*Kit Envision HRP Rabbit)* est appliqué sur les lames en chambre humide pendant 30 min à température ambiante. Après avoir rincées les lames dans du PBS (5 minutes) sous agitation, on applique la DAB (DAKO^{®}, Réf. : K 3466) sur les coupes pendant 2 à 5 minutes à température ambiante et en chambre humide. On rince les lames en renouvellement constant et on monte les lames sous la hotte chimique. L'analyse des résultats est réalisée à l'aide du logiciel ELLIX.

### - Marquage HPPS :

Des sections cryostat (8 µm) des muscles traités et témoins sont réalisés suivant les techniques standards. Les lames sont plongées dans l'hématoxyline de Harris pendant 3 minutes pour être ensuite rincées à l'eau courante. Les lames sont ensuite trempées dans l'alcool chlorhydrique, rincées et plongées dans l'eau de Scott pendant une minute. Après rinçage, les lames sont plongées dans la Phloxine pendant 30 secondes, rincées à l'eau courante et trempées dans l'éthanol absolu pendant une minute. Après une exposition au Safran pendant 3 minutes, les lames sont rincées à l'éthanol absolu et monté avec la résine Eukitt dont le solvant est le Xylène. L'analyse des résultats est réalisée à l'aide du logiciel CARTHOGRAPH.

### II) RESULTATS

### 1/ Poids des muscles à différents temps après injection dans des souris dystrophiques mdx :

Les muscles TAD et TAG ont été prélevés après 7 (J7), 14 (J14) ou 21 (J21) jours après l'injection, puis pesés. L'expérience a été répétée sur trois souris distinctes à chaque temps. Les résultats obtenus sont présentés dans les tableaux suivants :

**Temps J7 :**

| Souris | **Muscles** | **Poids (en g)** | **Croissance en % (100*TAG/TAD)-100** |
|---|---|---|---|
| **Souris 1** | **TAD 1** | 0,0661 | 3,18 |
| | **TAG1** | 0,0682 | |
| **Souris 2** | **TAD 2** | 0,0774 | 0,90 |
| | **TAG 2** | 0,0781 | |
| **Souris 3** | **TAD 3** | 0,0749 | 2,94 |
| | **TAG 3** | 0,0771 | |

**Temps J14 :**

| **Souris** | **Muscles** | **Poids** | **Croissance** |
|---|---|---|---|
| **Souris 4** | **TAD 4** | 0,0707 | 58,98 |
| | **TAG 4** | 0,1124 | |
| **Souris 5** | **TAD 5** | 0,0694 | 48,41 |
| | **TAG 5** | 0,103 | |
| **Souris 6** | **TAD 6** | 0,0854 | 6,67 |
| | **TAG 6** | 0,0911 | |

**Temps J21 :**

| **Souris** | **Muscles** | **Poids** | **Croissance** |
|---|---|---|---|
| **Souris 7** | **TAD 7** | 0,068 | 53,09 |
| | **TAG 7** | 0,1041 | |
| **Souris 8** | **TAD 8** | 0,0567 | 66,31 |
| | **TAG 8** | 0,0943 | |
| **Souris 9** | **TAD 9** | 0,0731 | 37,21 |
| | **TAG 9** | 0,1003 | |

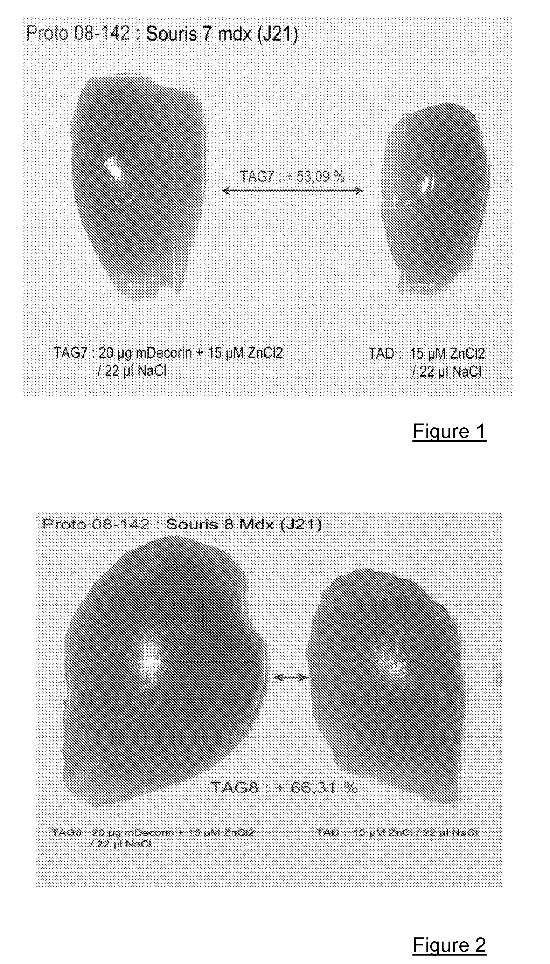

La différence de masse musculaire au jour 21 entre une souris *mdx* ayant ou non reçu une injection intramusculaire de décorine peut être visualisée aux Figures 1 et 2 concernant les souris 7 et 8, respectivement. On constate une nette augmentation de la masse musculaire (+ 53,09% et + 66,31%, respectivement).

### 2/ Poids des muscles à J18 après injection dans des souris dystrophiques gamma-sarcoglycan -l- :

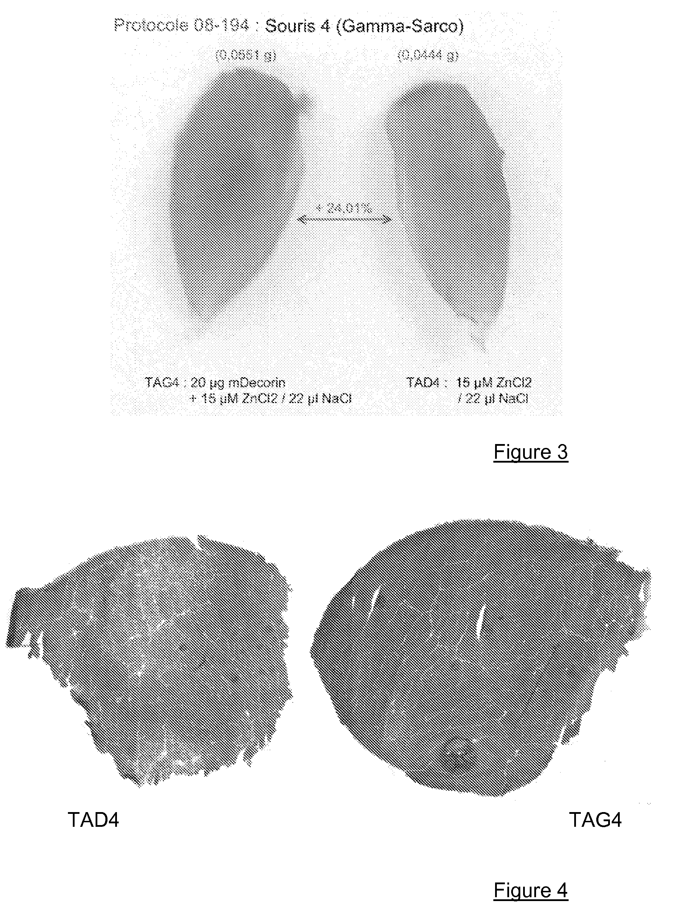

Une seconde série d'expériences a été réalisée sur 4 souris gamma-sarco -/-. Le protocole est identique à celui décrit pour *madx.* Les souris ont été sacrifiées au jour J18. Les résultats obtenus, illustrés aux figures 3 et 4, sont présentés dans le tableau suivant :

| **Souris** | **Muscles** | **Poids (en g)** | **Croissance** |
|---|---|---|---|
| **Souris 1** | **TAD 1** | 0,0456 | 10,75 |
| | **TAG 1** | 0,0505 | |
| **Souris 2** | **TAD 2** | 0,0413 | 17,43 |
| | **TAG 2** | 0,0485 | |
| **Souris 3** | **TAD 3** | 0,0528 | 12,31 |
| | **TAG 3** | 0,0593 | |
| **Souris 4** | **TAD 4** | 0,0444 | 24,10 |
| | **TAG 4** | 0,0551 | |

### 3/ Injection du peptide 31-71 issu de la partie N-terminale de la décorine murine dans des souris mdx :

Pour vérifier si la partie N-terminale de la décorine suffit pour observer des augmentations de masse musculaire, des expériences similaires ont été réalisées en présence du peptide mDCN 31-71 (SEQ ID NO : 7) correspondant aux résidus 31-71 de la décorine murine (SEQ ID NO : 6). Ce peptide a été décrit comme étant suffisant et nécessaire pour fixer le zinc (Yang VW, LaBrenz SR, Rosenberg LC, McQuillan D, Höök M. Decorin is a Zn2+ metalloprotein. J Biol Chem. 1999, 274(18):12454-60.).

Des souris *mdx* ont été injectées par voie IM dans le TA à l'aide des formulations suivantes :
TAG : 65 µg peptide 41 DCN + 15 µM ZnCl2 / 33 µl NaCl;
TAD : 15 µM ZnCl2 / 33 µl NaCl.

A J18, les souris ont été sacrifiées et le poids des muscles TAG et TAD a été mesuré. Les résultats sont présentés dans le tableau suivant :

| | ***Muscle*** | ***Poids en mg*** | ***Croissance*** |
|---|---|---|---|
| **Souris 4** | **TAD 4** | 53,6 | **8,77** |
| | **TAG 4** | 58,3 | |
| **Souris 5** | **TAD 5** | 39,2 | **19,39** |
| | **TAG 5** | 46,8 | |
| **Souris 6** | **TAD 6** | 40,1 | **24,69** |
| | **TAG 6** | 50 | |

Ces résultats montrent qu'un effet sur la croissance musculaire est conservé en présence de cette seule partie de la décorine.

Des résultats similaires ont pu être obtenus avec un peptide encore plus court de 30 acides aminés, présentant la séquence SEQ ID NO : 15.

## Revendications

1. Composition comprenant un fragment de la décorine apte à fixer le zinc de moins de 100 acides aminés pour son utilisation dans le traitement des pathologies associées à une fonte musculaire.

2. Composition pour son utilisation selon la revendication 1, ***caractérisée* en ce que** les pathologies sont choisies dans le groupe comprenant : les maladies neuromusculaires, avantageusement les dystrophies musculaires telles que la myopathie de Duchenne, et les cachexies.

3. Utilisation non thérapeutique d'une composition comprenant un fragment de la décorine apte à fixer le zinc de moins de 100 acides aminés pour l'augmentation de la masse musculaire.

4. Utilisation selon la revendication 3, ***caractérisée* en ce que** l'augmentation de la masse musculaire vise à compenser la fonte résultant d'une immobilisation ou de la vieillesse.

5. Utilisation selon la revendication 3 ou 4, ***caractérisée* en ce qu'**elle est destinée à l'animal.

6. Composition pour son utilisation selon la revendication 1 ou 2 ou utilisation selon l'une des revendications 3 à 5, ***caractérisée* en ce que** le fragment comprend la séquence SEQ ID NO: 7, 15 ou 16.

7. Composition pour son utilisation selon la revendication 1 ou 2 ou utilisation selon l'une des revendications 3 à 5, ***caractérisée* en ce que** le fragment présente la séquence SEQ ID NO : 7, 15 ou 16.

8. Composition pour son utilisation selon la revendication 1 ou 2 ou utilisation selon l'une des revendications 3 à 5, ***caractérisée* en ce que** le fragment est un fragment d'une protéine recombinante ou d'une protéine fusion, ou se présente sous la forme d'un acide nucléique codant un tel fragment.

9. Composition pour son utilisation selon la revendication 1 ou 2 ou utilisation selon l'une des revendications 3 à 5, ***caractérisée* en ce que** la composition est destinée à être injectée sous forme intramusculaire, intrapéritonéale ou intraveineuse.

10. Composition pour son utilisation selon la revendication 1 ou 2, ***caractérisée* en ce que** la composition est associée à d'autres traitements, notamment la thérapie génique et la greffe de cellules.

## Patentansprüche

1. Zusammensetzung umfassend ein Fragment von Decorin, das in der Lage ist Zink zu fixieren, mit weniger als 100 Aminosäuren, zur Verwendung in der Behandlung von Pathologien, die mit Muskelschwund verbunden sind.

2. Zusammensetzung zur Verwendung gemäß Anspruch 1, ***dadurch gekennzeichnet, dass*** diese Pathologien zu der Gruppe gehören, die die neuromuskulären Krankheiten, vorzugsweise Muskeldystrophien, wie die Duchenne-Muskeldystrophie und Kachexien, umfasst.

3. Nicht-therapeutische Verwendung einer Zusammensetzung, umfassend ein Fragment von Decorin, das in der Lage ist Zink zu fixieren, mit weniger als 100 Aminosäuren, zur Erhöhung der Muskelmasse.

4. Verwendung gemäß Anspruch 3, ***dadurch gekennzeichnet, dass*** die Erhöhung der Muskelmasse darauf abzielt, den Muskelschwund auszugleichen, der sich durch eine Immobilisierung oder das Alter ergibt.

5. Verwendung gemäß Anspruch 3 oder 4, ***dadurch gekennzeichnet, dass*** es für Tiere bestimmt ist.

6. Zusammensetzung zur Verwendung gemäß Anspruch 1 oder 2, oder Verwendung nach einem der Ansprüche 3 bis 5, ***dadurch gekennzeichnet, dass*** das Fragment die Sequenz SEQ ID NO: 7, 15 oder 16 umfasst.

7. Zusammensetzung zur Verwendung gemäß Anspruch 1 oder 2, oder Verwendung nach einem der Ansprüche 3 bis 5, ***dadurch gekennzeichnet, dass*** das Fragment die Sequenz SEQ ID NO: 7, 15 oder 16 hat.

8. Zusammensetzung zur Verwendung gemäß Anspruch 1 oder 2, oder Verwendung nach einem der Ansprüche 3 bis 5, *dadurch gekennzeichet, dass* das Fragment das Fragment eines rekombinanten Proteins oder eines Fusionsproteins ist oder in Form einer ein solches Fragment kodierenden Nukleinsäure vorliegt.

9. Zusammensetzung zur Verwendung gemäß Anspruch 1 oder 2, oder Verwendung nach einem der Ansprüche 3 bis 5, ***dadurch gekennzeichnet, dass*** die Zusammensetzung in intramuskulärer, intraperitonealer oder intravenöser Form injiziert werden soll.

10. Zusammensetzung zur Verwendung gemäß Anspruch 1 oder 2, ***dadurch gekennzeichnet, dass*** die Zusammensetzung mit anderen Behandlungen kombiniert wird, vor allem Gentherapie und Zelltransplantation.

## Claims

1. Composition containing a fragment of decorin able to bind zinc of less than 100 amino acids for use to treat diseases associated with muscle wasting.

2. Composition for its use according to claim 1, ***characterised* in that** the diseases are selected from the group comprising: neuromuscular diseases, to advantage muscular dystrophies such as Duchenne muscular dystrophy, and the cachexia.

3. Non therapeutic use of a composition containing a fragment of decorin able to bind zinc of less than 100 amino acids to increase muscle mass.

4. Use according to claim 3, ***characterised* in that** the aim of increasing muscle mass is to compensate for wasting resulting from immobilisation or old age.

5. Use according to claim 3 or 4, ***characterised* in that** it is for use in animals.

6. Composition for its use according to claim 1 or 2 or use according to claims 3 to 5, ***characterised* in that** the fragment comprises the sequence SEQ ID NO: 7, 15 or 16.

7. Composition for its use according to claim 1 or 2 or use according to claims 3 to 5, ***characterised* in that** the fragment has the sequence SEQ ID NO: 7, 15 or 16.

8. Composition for its use according to claim 1 or 2 or use according to claims 3 to 5, ***characterised* in that** the fragment is a fragment of a recombinant protein or a fusion protein, or is in the form of a nucleic acid encoding such a fragment.

9. Composition for its use according to claim 1 or 2 or use according to claims 3 to 5, ***characterised* in that** the composition is for intramuscular, intraperitoneal or intravenous injection.

10. Composition for its use according to claim 1 or 2, ***characterised* in that** the composition is associated with other treatments, particularly gene therapy and cell grafting.
